# EUROPEAN PATENT SPECIFICATION

(11) **EP 4 586 979 B1**
(45) Date of publication and mention of the grant of the patent: **13.05.2026**
(21) Application number: 24775994.7
(22) Date of filing: 23.08.2024
(51) Int. Cl.: A61F 13/00, A61B 17/00, A61F 13/02, A61F 13/0246, A61K 9/70, A61L 15/44

(54) **WOUND CLOSURE SYSTEM HAVING MEDICANT**
WUNDVERSCHLUSSSYSTEM MIT MEDIKAMENT
SYSTÈME DE FERMETURE DE PLAIE AYANT UN MÉDICAMENT

(30) Priority: 23.08.2023 US 202318454270
(43) Date of publication of application: 23.07.2025
(73) Proprietor: Cilag GmbH International, 6300 Zug (CH)
(72) Inventor: OU, Duan Li, Raritan, New Jersey 08869 (US); TANNHAUSER, Robert J., Raritan, New Jersey 08869 (US); KRIKSUNOV, Leo, Raritan, New Jersey 08869 (US)
(74) Representative: Carpmaels & Ransford LLP
(86) International application number: PCT/IB2024/058219
(87) International publication number: WO 2025/041094

(56) References cited:
- WO-A1-2021/080867
- US-A1- 2023 158 284

## Description

### BACKGROUND

A wound closure system (also referred to as a skin closure system) may be used at the conclusion of a surgical procedure on a patient to close a wound (e.g., a surgical incision) that was formed in the patient's skin for accessing a target anatomical structure. By way of example, wound closure systems may include components such as sutures, substrates, and/or liquid topical skin adhesives that are applied by a surgeon to approximate the edges of the wound and, in some cases, form a stable and protective layer over the wound that promotes efficient healing. In some instances, one or more components of the applied wound closure system may be absorbed by the patient during the healing process. Following healing of the wound, remaining components of the wound closure system may be removed from the skin by a surgeon, and/or they may automatically separate from the skin such that they may be discarded by the patient.

WO2021/080867A1 describes elastic therapeutic tapes which are adapted for adhesive application to the external skin of a mammal. US2023/158284A1 describes wound dressings which can generate nitric oxide.

While various wound closure systems and associated components and methods have been made and used, it is believed that no one prior to the inventors has made or used the invention described in the appended claims.

### BRIEF DESCRIPTION OF THE DRAWINGS

The accompanying drawings, which are incorporated in and constitute a part of this specification, illustrate embodiments of the invention, and, together with the description given below, serve to explain the principles of the present invention.
FIG. 1 depicts a perspective view of an example of a wound closure system that includes a wound closure device, an adhesive applicator, and an adhesive spreader;
FIG. 2 depicts a disassembled perspective view of the wound closure device of FIG. 1, showing a mesh layer, a pressure sensitive adhesive layer, and a removable backing layer;
FIG. 3A depicts a perspective view of the wound closure device of FIG. 1 aligned longitudinally with a wound in the skin of a patient, showing a central section of the backing layer having been removed;
FIG. 3B depicts a perspective view of the wound closure device of FIG. 1 applied to the patient's skin over the wound to approximate the edges of the wound;
FIG. 3C depicts a perspective view of the wound closure device of FIG. 1 applied to the patient's skin over the wound, showing remaining sections of the backing layer having been removed so the wound closure device is fully adhered to the skin;
FIG. 3D depicts a perspective view of the wound closure device of FIG. 1 applied to the patient's skin over the wound, showing application of a liquid topical skin adhesive onto the mesh layer with the adhesive applicator;
FIG. 3E depicts a perspective view of the wound closure device of FIG. 1 applied to the patient's skin over the wound, showing the adhesive spreader positioned against the patient at the start of an adhesive spreading stroke for spreading the applied topical skin adhesive over and through the wound closure device;
FIG. 3F depicts a perspective view of the wound closure device of FIG. 1 applied to the patient's skin over the wound, showing the adhesive spreader during a subsequent portion of the adhesive spreading stroke;
FIG. 4 depicts a perspective view of an example of a wound closure system that includes the wound closure device of FIG. 1, the adhesive applicator of FIG. 1, the adhesive spreader of FIG. 1, and a localized active strip having an active therapeutic agent incorporated therein, the localized active strip being housed within a storage container;
FIG. 5 depicts a perspective view of the localized active strip and storage container of FIG. 4, with the localized active strip being removed from the storage container;
FIG. 6A depicts a perspective view of the localized active strip of FIG. 4 aligned longitudinally with a wound in the skin of a patient;
FIG. 6B depicts a perspective view of the localized active strip of FIG. 4 applied to the patient's skin over the wound;
FIG. 6C depicts a perspective view of the wound closure device of FIG. 1 aligned longitudinally with both the localized active strip of FIG. 4 and the wound in the skin of a patient, showing a central section of the backing layer having been removed;
FIG. 6D depicts a perspective view of the wound closure device of FIG. 1 applied to the patient's skin over the wound and the localized active strip of FIG. 4, showing remaining sections of the backing layer having been removed so the wound closure device is fully adhered to the skin;
FIG. 6E depicts a perspective view of the wound closure device of FIG. 1 applied to the patient's skin over the wound and the localized active strip of FIG. 4, showing application of a liquid topical skin adhesive onto the mesh layer with the adhesive applicator;
FIG. 6F depicts a perspective view of the wound closure device of FIG. 1 applied to the patient's skin over the wound and the localized active strip of FIG. 4, showing the adhesive spreader during a subsequent portion of the adhesive spreading stroke;
FIG. 6G depicts a perspective view of the wound closure device of FIG. 1 applied to the patient's skin over the wound and the localized active strip of FIG. 4;
FIG. 7 depicts a perspective view of an alternative wound closure device and a localized active strip, where the localized active strip is attached to the underside of the wound closure device;
FIG. 8 depicts a cross-sectional view, taken along line 8-8 of FIG. 6G, of the wound closure device of FIG. 1 and the localized active strip of FIG. 4 applied to the patient skin, with the localized active strip being directly adjacent to the wound of the patient;
FIG. 9 depicts a cross-sectional view of an alternative localized active strip and the wound closure device of FIG. 1 applied to the patient skin, with the localized active strip being directly adjacent to the wound of the patient;
FIG. 10A depicts a cross-sectional view of an alternative localized active strip and the wound closure device of FIG. 1 applied to the patient skin, with pressure sensitive adhesive of the localized active strip being directly adjacent to the wound of the patient; and
FIG. 10B depicts a cross-sectional view of the localized active strip of FIG. 10A and the wound closure device of FIG. 1 applied to the patient skin, with the pressure sensitive adhesive of the localized active strip dissolved.

The drawings are not intended to be limiting in any way, and it is contemplated that various embodiments of the invention may be carried out in a variety of other ways, including those not necessarily depicted in the drawings. The accompanying drawings incorporated in and forming a part of the specification illustrate several aspects of the present invention, and together with the description serve to explain the principles of the invention; it being understood, however, that this invention is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

For clarity of disclosure, the terms "proximal" and "distal" are defined herein relative to a human or robotic operator of the surgical instrument. The term "proximal" refers to the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. It will be further appreciated that, for convenience and clarity, spatial terms such as "side," "upwardly," and "downwardly" also are used herein for reference to relative positions and directions. Such terms are used below with reference to views as illustrated for clarity and are not intended to limit the invention described herein.

Furthermore, the terms "about," "approximately," and the like as used herein in connection with any numerical values or ranges of values are intended to encompass the exact value(s) referenced as well as a suitable tolerance that enables the referenced feature or combination of features to function for the intended purpose described herein.

### I. Wound Closure System

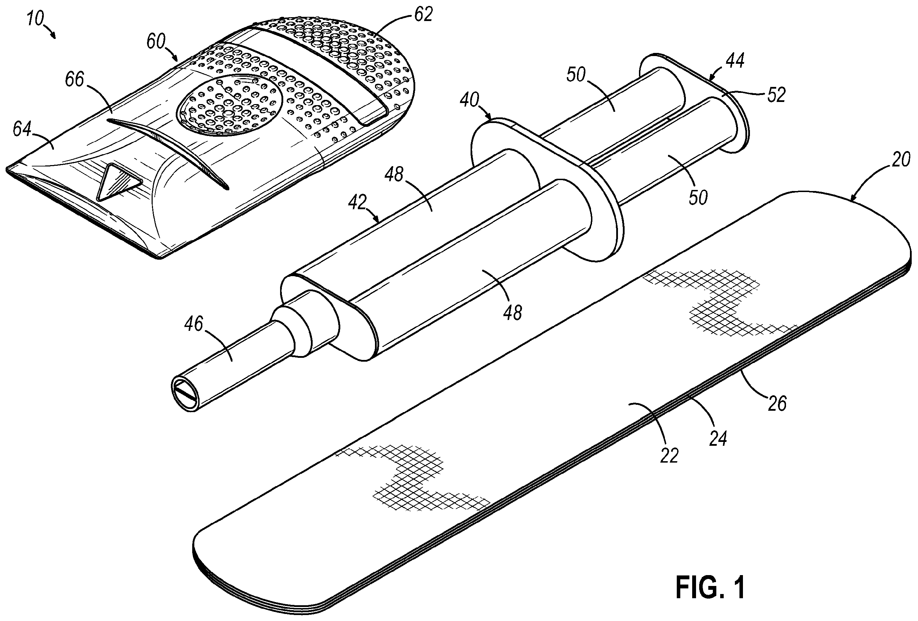

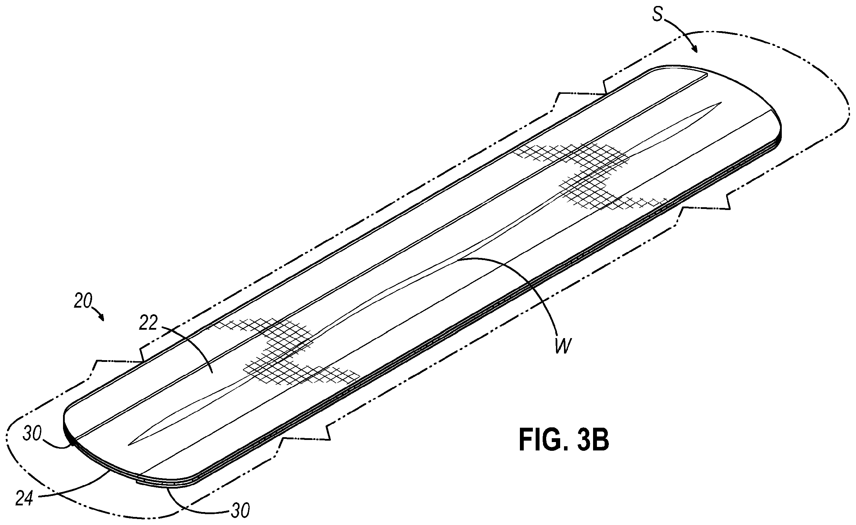

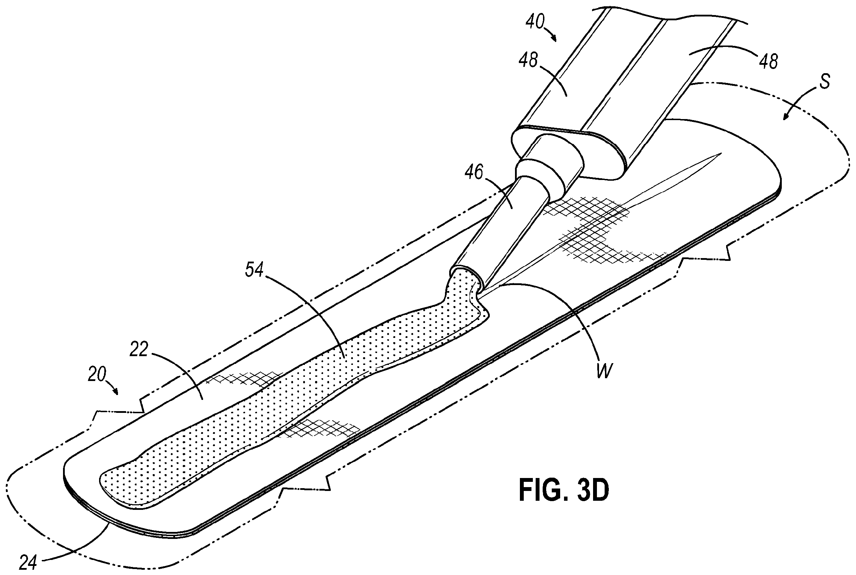

FIG. 1 shows an example of a wound closure system (10) that includes a wound closure device (20) (also referred to as a patch) configured to be applied to a patient's wound (W), an adhesive applicator (40) configured to apply a topical skin adhesive (54) (see FIG. 3D) to the applied wound closure device (20), and an adhesive spreader (60) configured to spread the applied topical skin adhesive (54) onto and through wound closure device (20). Each of these components is described in greater detail below.

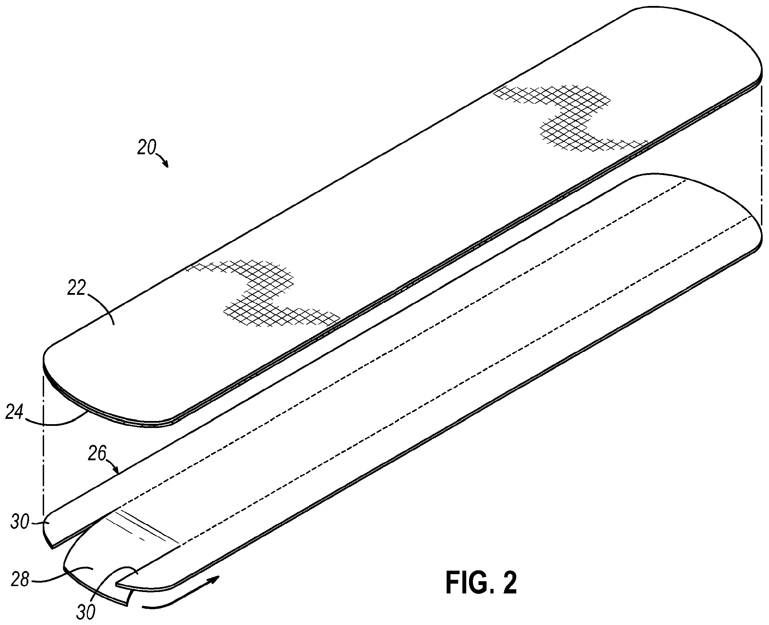

As shown best in FIG. 2, wound closure device (20) of the present version has an elongate, generally rectangular shape and a triple layer construction. More specifically, wound closure device (20) includes a layer of substrate in the form of a textile mesh (22), a layer of pressure sensitive adhesive (24) formed as a continuous or non-continuous coating along the lower skin-facing side of mesh (22), and a layer of backing (26) removably applied to the lower side of pressure sensitive adhesive (24). It will be understood that the term "wound closure device" as used herein encompasses wound closure device (20) both with and without backing (26), which may be removed and discarded during application of wound closure device (20) to a patient, as described in greater detail below.

Mesh (22) is configured to retain a liquid topical skin adhesive and may be formed of polyethylene terephthalate (PET) or any other suitable surgical textile material. Pressure sensitive adhesive (24) is configured to enable wound closure device (20) to self-adhere to a patient's skin in response to a pressure being applied to the upper side of mesh (22) during its application by a surgeon. Backing (26) serves to protect pressure sensitive adhesive (24) before application of wound closure device (20) to the patient. In the present version, backing (26) includes elongate arrays of perforations that extend longitudinally and define an elongate central backing section (28) and a pair of elongate side backing sections (30). Though each backing section (28, 30) is shown as generally rectangular in the present version, backing sections (28, 30) may be of various alternative shapes, sizes, and quantities in other versions.

As shown in FIG. 1, adhesive applicator (40) includes an applicator body (42), a plunger unit (44) slidably received within an open proximal end of applicator body (42), and a static mixer (46) secured to a distal end of applicator body (42). Applicator body (42) includes a pair of barrels (48) arranged side by side, where each barrel (48) houses a respective part of a two-part liquid topical skin adhesive. Plunger unit (44) includes a pair of plungers (50) that are arranged side by side and are interconnected at their proximal ends by a bridge (52). Each plunger (50) is actuatable distally through a respective barrel (48) of applicator body (42) to force the corresponding liquid adhesive part distally into static mixer (46). Static mixer (46) is configured to receive the first and second adhesive parts and direct them around and through a series of static baffles and passages (not shown) that mix the two adhesive parts together into a homogenous liquid adhesive (54) that is then dispensed through an open distal end of static mixer (46), as shown in FIG. 3D described below.

In the present version, liquid topical skin adhesive (54) is in the form of a silicone-based topical skin adhesive that is configured to cure on skin at body temperature in less than two minutes. Once cured, topical skin adhesive (54) remains elastomeric such that a given section of cured adhesive (54) is configured to stretch up to 160% of its cured length and then fully recover to the cured length. Accordingly, wound closure system (10) may be particularly effective for use on actuatable body parts of a patient such as a knee, wrist, elbow, or other joint, for example.

As also shown in FIG. 1, adhesive spreader (60) of the present version has a monolithic body that includes a proximal body portion (62) configured to be gripped by a user, a distal body portion (64) that terminates at a tip configured to spread applied topical skin adhesive (54), and an intermediate flexural body portion (66) that interconnects the proximal and distal body portions (62, 64). Flexural body portion (66) is configured to elastically deform so that distal body portion (64) angularly deflects relative to proximal body portion (62) to promote effective spreading of topical skin adhesive (54) along wound closure device (20) with a suitable normal force within a predetermined range.

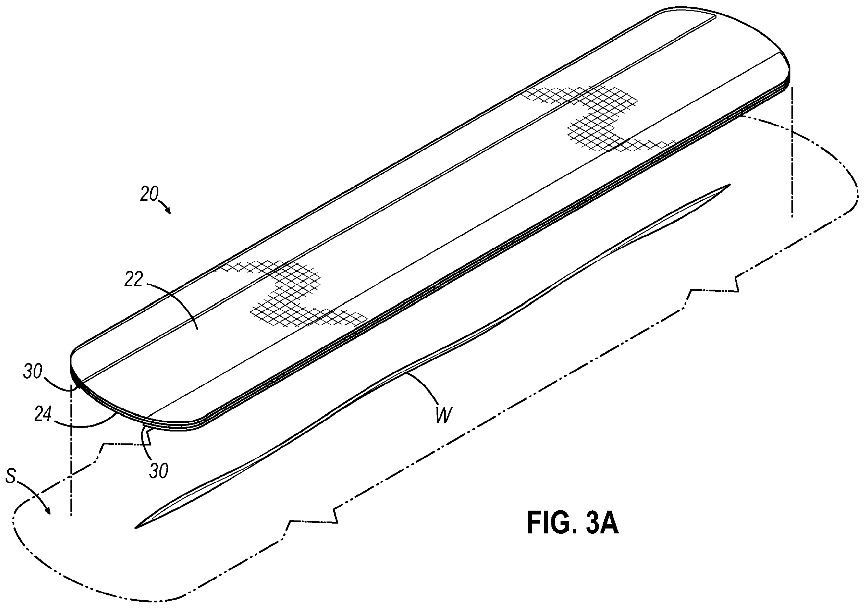

FIGS. 3A-3D show an example of wound closure system (10) being used to close a wound (W) formed in the skin (S) of a patient. In some procedures, wound (W) may be at least partially closed with one or more sutures, for example at deeper portions of wound (W), prior to use of wound closure system (10). Additionally, before wound closure device (20) is applied to the patient, it may be trimmed by a surgeon to any suitable shape as desired.

As shown in FIG. 3A, central backing section (28) is removed from wound closure device (20) to expose a central window of mesh (22) and pressure sensitive adhesive (24), and an imaginary centerline of wound closure device (20) is aligned longitudinally with the edges of wound (W). Wound closure device (20) is then applied to the patient skin (S) over wound (W) and the surgeon applies pressure to the central portion of mesh (22) to force pressure sensitive adhesive (24) to adhere to the skin (S), thus fixing the edges of wound (W) relative to one another. Before this step, the edges of wound (W) may be held in an approximated state by the surgeon. Alternatively, during this step the central portion of wound closure device (20) may be applied in a laterally alternating manner to approximate the edges of wound (W) during application. With either approach, wound closure device (20) is configured to hold the edges of wound (W) in an approximated state following this initial step of application. Once the surgeon is satisfied with the position of wound closure device (20) relative to wound (W), the surgeon may then remove the remaining two side backing sections (30) to adhere the reminder of wound closure device (20) to skin (S).

As shown in FIG. 3D, adhesive applicator (40) is then used to apply a pattern of topical skin adhesive (54) to the upper side of mesh (22) of the applied wound closure device (20). While adhesive applicator (40) is shown applying a linear bead of topical skin adhesive (54) in the present version, it will be appreciated that various other patterns of topical skin adhesive (54) may be applied in other versions, such as a T-shaped pattern or a wave-shaped pattern, as described in greater detail below.

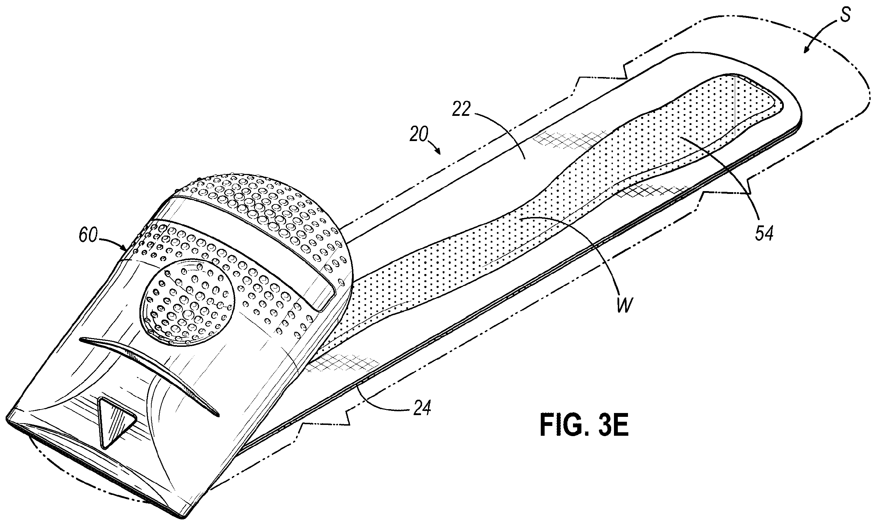

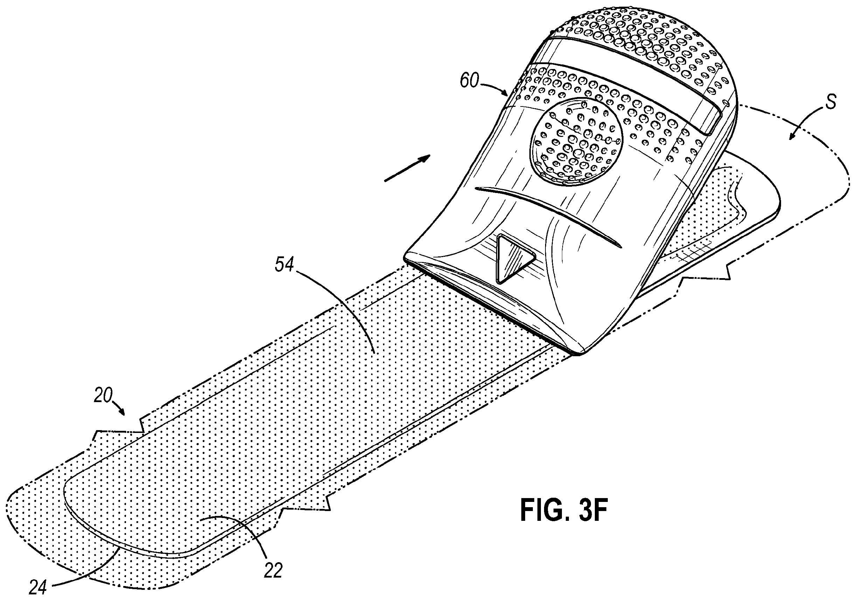

As shown in FIGS. 3E-3F, adhesive spreader (60) is then used to spread the applied topical skin adhesive (54) uniformly over wound closure device (20) to force the topical skin adhesive (54) through the layers of mesh (22) and pressure sensitive adhesive (24) and directly against wound (W) and the surrounding skin (S). In that regard, the layers of mesh (22) and pressure sensitive adhesive (24) may be at least partially permeable to permit forced passage of topical skin adhesive (54) therethrough. As shown in FIG. 3E, flexural body portion (66) of adhesive spreader (60) may be in a non-deformed state when adhesive spreader (60) is first positioned against wound closure device (20) at the beginning of an adhesive spreading stroke. As adhesive spreader (60) is dragged longitudinally along wound closure device (20), the input force exerted by the user may cause flexural body portion (66) to elastically deform such that distal body portion (64) angularly deflects relative to proximal body portion (62), as shown in FIG. 3F. The degree of deformation of flexural body portion (66) may be directly related to the viscosity of topical skin adhesive (54). In particular, a greater viscosity may require that the user exert a greater input force through proximal body portion (62) to effectively spread topical skin adhesive (54) over and through wound closure device (20), such that the flexural body portion (66) deforms a relatively greater amount. Conversely, a lesser viscosity may require a lesser input force such that the flexural body portion (66) deforms less or not at all.

Optionally, topical skin adhesive (54) may also be spread over adjacent portions of skin (S) not covered by wound closure device (20) to ensure that an entirety of mesh (22) is embedded with topical skin adhesive (54). For instance, and by way of example only, topical skin adhesive (54) may be spread onto at least 1 cm of skin (S) about the entire outer perimeter of the applied wound closure device (20). Once topical skin adhesive (54) has been fully spread over wound closure device (20), any topical skin adhesive (54) on skin (S) beyond the perimeter of device (20) may then be wiped away with sterile gauze, for example. Additionally, in some instances, a quantity of topical skin adhesive (54) may be applied between the edges of wound (W) before wound closure device (20) is applied to the skin (S). The applied topical skin adhesive (54) is then cured within and over wound closure device (20) to form a composite microbial barrier over wound (W) that maintains a protective environment that promotes efficient healing. Following healing of wound (W), wound closure device (20) may be removed from the skin (S) manually (e.g., by a surgeon) or it may automatically separate from the skin (S) such that it may be discarded by the patient.

Wound closure system (10) may be further configured and operable in accordance with at least some of the teachings of U.S. Pat. Pub. No. 2021/0369258, entitled "Systems, Devices and Methods for Dispensing and Curing Silicone Based Topical Skin Adhesives," published December 2, 2021; U.S. Pat. Pub. No. 2021/0371190, entitled "Systems, Methods and Devices for Aerosol Spraying of Silicone Based Topical Skin Adhesives for Sealing Wounds," published December 2, 2021; U.S. Pat. Pub. No. 2021/0371658, entitled "Novel Topical Skin Closure Compositions and Systems," published December 2, 2021; U.S. Pat. Pub. No. 2021/0369639, entitled "Novel Antimicrobial Topical Skin Closure Compositions and Systems," published December 2, 2021; U.S. Pat. Pub. No. 2021/0369276, entitled "Anisotropic Wound Closure Systems," published December 2, 2021; U.S. Pat. App. No. 17/667,950, entitled "Gas Sterilizable Syringes Having Apertures Covered by Gas Permeable Barriers for Enabling Ingress and Egress of Sterilization Gases While Preventing Leakage of Flowable Materials," filed on February 9, 2022; U.S. 17/991,992, entitled "Application of Topical Skin Adhesive to Surgical Mesh," filed on November 22, 2022; U.S. 17,991,945, entitled "Surgical Mesh Securing Device For Wound Closure System," filed on November 22, 2022; and/or U.S. 17/991,950, entitled "Wound Closure System Having Microcannulaic Pathways," filed on November 22, 2022.

### II. Illustrative Wound Closure Systems with Medicant

As mentioned above, wound closure device (20) is configured to be applied to a patient's wound (W) while topical skin adhesive (54) is configured to cure within and over wound closure device (20). Together, wound closure device (20) and cured adhesive (54) form a composite microbial barrier over wound (W); while cured adhesive (54) is suitably elastomeric such that a given section of cured adhesive (54) may stretch and fully recover to its cured length. In some instances, it may be desirable to apply an active pharmaceutical ingredient (i.e., an "API") directly onto and/or into the wound (W) while wound closure device (20) and topical skin adhesive (54) form the composite microbial barrier over wound (W).

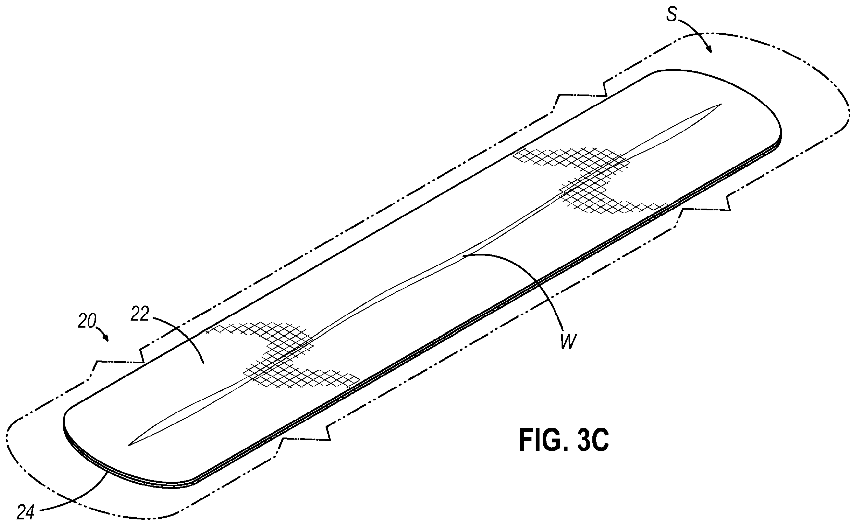

However, in instances where an API is directly incorporated into mesh (22) of wound closure device (20) and/or topical skin adhesive (54) (e.g., an API impregnated into mesh (22)), the API may be located too great a distance from the wound (W) such that a minimum amount API necessary to provide a desired therapeutic effect is unable to timely migrate or diffuse from its applied location to wound (W), in which case the API may fail to provide the desired therapeutic effect. For example, as shown in FIGS. 3C-3D, the lateral edges of mesh (22) may be located too far from wound (W) to enable corresponding API associated with lateral edges of mesh (220) to suitably reach wound (W) and provide the desired therapeutic effect. Therefore, it may be desirable to utilize a wound closure device (20) that applies a suitable amount of API directly onto/into a wound (W) covered by a microbial barrier to ensure that the intended therapeutic effect of the API is achieved.

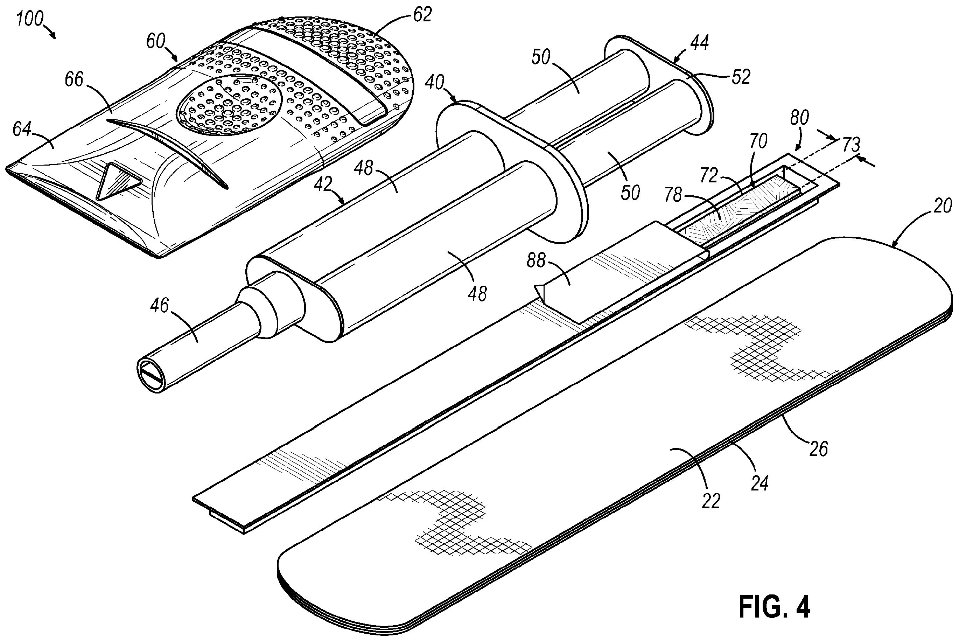

FIG. 4 shows an illustrative alternative wound closure system (100) that is configured in such a manner and may be used in replacement of wound closure system (10) described above. Wound closure system (100) is substantially similar to wound closure system (10) described above, with differences elaborated herein. Wound closure system (100) includes wound closure device (20) (i.e., a patch), adhesive applicator (40) configured to apply topical skin adhesive (54) onto patch (20), and adhesive spreader (60) configured to spread topical skin adhesive (54) on top of, and into, patch (20).

Additionally, as best shown in FIGS. 4-5, wound closure system (100) includes a localized active strip (70). As will be described in greater detail below, localized active strip (70) is impregnated with a suitable API (78), such as triclosan, that is configured to migrate and/or diffuse off localized active strip (70). As will also be described in greater detail below, localized active strip (70) is suitably narrow such that API (78) located at lateral edges of strip (70) may suitably migrate and/or diffuse toward wound (W) within a suitable time frame to achieve the desired therapeutic effect as wound (W) heals.

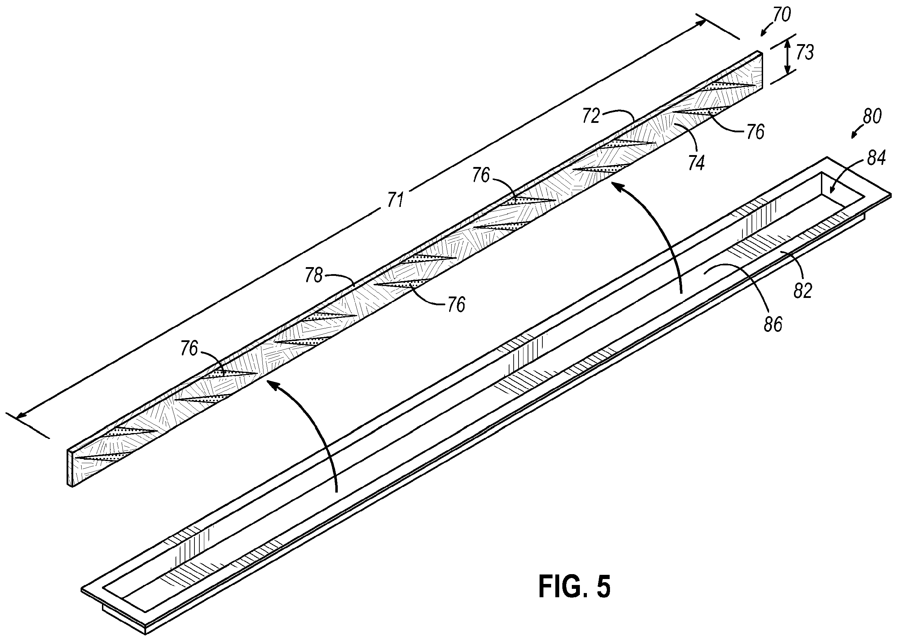

As best shown in FIG. 5, localized active strip (70) includes a narrow, elongated body (72) having an underside (74) with pressure sensitive adhesive (76) attached thereto. In the current example, elongated body (72) is formed of a suitable surgical mesh material, such as polyethylene terephthalate (PET) or any other suitable surgical textile material. However, elongated body (72) may be formed of any suitable material as would be apparent to one skilled in the art in view of the teachings herein. In some instances, elongated body (72) has a lower porosity compared to that of mesh (22). In some instances, elongated body (72) if formed of a material that is impervious to penetration by topical skin adhesive (54), unlike mesh (22) described above. As will be described in greater detail below, elongated body (72) may be configured to inhibit, at least to some degree, topical skin adhesives (54) from penetrating through elongated body (72) and cure between the underside (74) of strip (70) and skin (S)/wound (W) of the patient.

Elongated body (72) is impregnated with a suitable amount of API (78). API (78) is associated with elongated body (72) such that after localized active strip (70) is adhered to a patient in accordance with the description herein, API (78) migrates and/or diffuses off elongated body (72). In some instances, exposure to moisture, such as bodily fluids, may encourage API (78) to migrate and/or diffuse off elongated body (72). In some instances, exposure to a temperature above or below a threshold temperature may encourage API (78) to migrate and/or diffuse off elongated body (72). Any suitable API (78) may be incorporated into elongated body (72) as would be apparent to one skilled in the art in view of the teachings herein. As one example, triclosan may be incorporated into the mesh forming elongated body (72).

Pressure sensitive adhesive (76) is substantially similar to pressure sensitive adhesive (24) of wound closure device (20). Therefore, pressure sensitive adhesive (76) is configured to enable active strip (70) to self-adhere to a patient skin in response to a pressure being applied to underside (74) of elongated body (72) during application by a surgeon. While not shown, it should be understood that a removable backing, similar to backing (28, 30) described above, may be attached to underside (74) to protect pressure sensitive adhesive (76). Pressure sensitive adhesive (76) is disposed on underside (74) of elongated body (72) in a "tiger stripe" pattern. Such a tiger stripe pattern may allow active strip (70) to self-adhere to a patient skin, while also exposing portions of underside (74) directly to wound (W) after strip (70) is suitably applied on top of wound (W). Pressure sensitive adhesive (76) may be disposed on underside (74) of elongated body (72) in any suitable geometry as would be apparent to one skilled in the art in view of the teachings herein. As will be described in greater detail below, in some instances, pressure sensitive adhesive (76) may be configured to dissolve after strip (70) is suitably applied to wound (W) and a microbial barrier is formed, thereby exposing underside (74) to wound (W) for migration/disbursement of API (78) into wound (W).

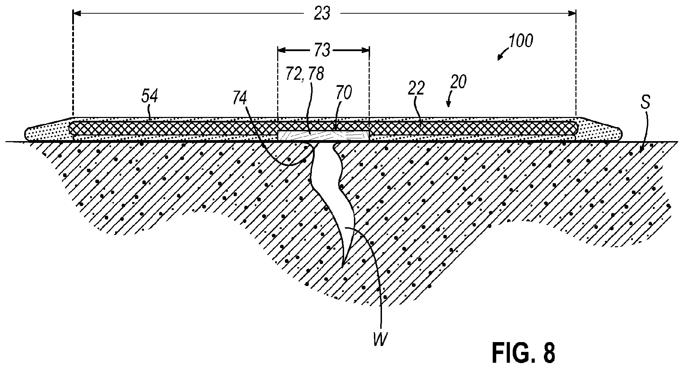

Elongated body (72) includes a suitable length (71) dimensioned to span over the length of a wound (W). Length (71) may be modified during illustrative use by a surgeon in order to customize the length (71) to accommodate the length of wound (W). In some instances, elongated body (72) may come in a roll, such that length (71) may be customized for each wound (W). Elongated body (72) also includes a suitable width (73) configured to span over the width of the wound (W). However, as mentioned above, width (73) of elongated body (72) is suitably narrow such that, after application of elongated body (72) in accordance with the description herein, API (78) located at lateral edges may suitably migrate and/or diffuse toward wound (W) within a suitable time frame to achieve the desired therapeutic effect as wound (W) heals. As best shown in FIG. 8, width (73) of active strip (70) is substantially narrower than width (23) of wound closure device (20). In some instances, width (73) of elongated body (72) is around or between 1/8 to 1/2 of the width (23) of wound closure device (20). With a narrower width (73) compared to width (23) of wound closure device (20), that area of underside (74) may be around 5% to about 30% compared to the area of the underside of mesh (22). Therefore, wound closure device (20) may be more suitable for approximating the edges of wound (W); while active strip (70) is more suitable for concentrating/localizing API (78) directly adjacent to wound (W).

Any suitable methods and devices may be utilized to incorporate API (78) into the surgical mesh structure of elongated body (72). For example, the API may be deposited onto the surgical mesh body (72) through an ethylene oxide sterilization process. In instances where API (78) comprises triclosan, the triclosan may be deposited onto the surgical mesh body (72) through an ethylene oxide sterilization process. More specifically, the triclosan may first be incorporated into a source reservoir comprising a triclosan silicone composite that includes a low temperature curable liquid silicone rubber, for example as disclosed in U.S. Pat. Pub. No US2025065010A1, entitled "Low Temperature Curable Liquid Silicone Rubber Carrier for Active Pharmaceutical Ingredient," filed on even date herewith. The triclosan-containing source reservoir and surgical mesh body (72) are then positioned within a seal pouch, which is placed into an ethylene oxide chamber to initiate an ethylene oxide sterilization process during which heat and vacuum are applied. During this sterilization process, the triclosan migrates from the source reservoir into the surgical mesh body (72), resulting in the formation of triclosan crystals on the mesh structure of elongated body (72). While triclosan is utilized in the current example, any other suitable API may be utilized as would be apparent to one skilled in the art in view of the teachings herein.

In some instances, API (78) associated with elongated body (72) may be volatile in the sense that API (78) may attempt to migrate off elongated body (72) prior to illustrative use. Therefore, prior to illustrative use of localized active strip (70) in accordance with the description herein, strip (70) may be stored within a container (80) having a sealed, peelable cover (88). Container (80) includes an elongated body (82) having an interior surface (86) defining a recessed area (84). Active strip (70) is dimensioned to fit within recessed area (84) defined by interior surface (86). Additionally, prior to illustrative use, peelable cover (88) may suitably attach to a perimeter of elongated body (82) defining recessed area (84) while active strip (70) is housed within recessed area (84), thereby creating a fluid tight seal between active strip (70) housed within recessed area (84) and the external environment. Isolating active strip (70) within container (80) may inhibit a volatile API (78) from undesirably migrating off active strip (70) prior to illustrative use in accordance with the description herein. Once a surgeon desires to utilize active strip (70), peelable cover (88) may be removed, thereby exposing active strip (70) to the external environment and allowing a surgeon to remove strip (70) from container (80) for illustrative use in accordance with the description herein. While elongated body (72) and peelable cover (88) are used to store active strip (70) in the current example, any other suitable structures may be used as would be apparent to one skilled in the art in view of the teachings herein. For example, a sealable bag may be utilized to store active strip (70).

Alternatively, or in addition to storing active strip (70), elongated body (82) may be formed of a low temperature cured triclosan silicone composite described above such that active strip (70) may be placed in recessed area (84) of container (80) during a sterilization process in order to incorporate triclosan onto active strip (70) in accordance with the description herein. Therefore, in some instances, container (80) may be used to house active strip (70) prior to illustrative use; and/or container (80) may be used to apply triclosan onto active strip (70) in preparation of illustrative use.

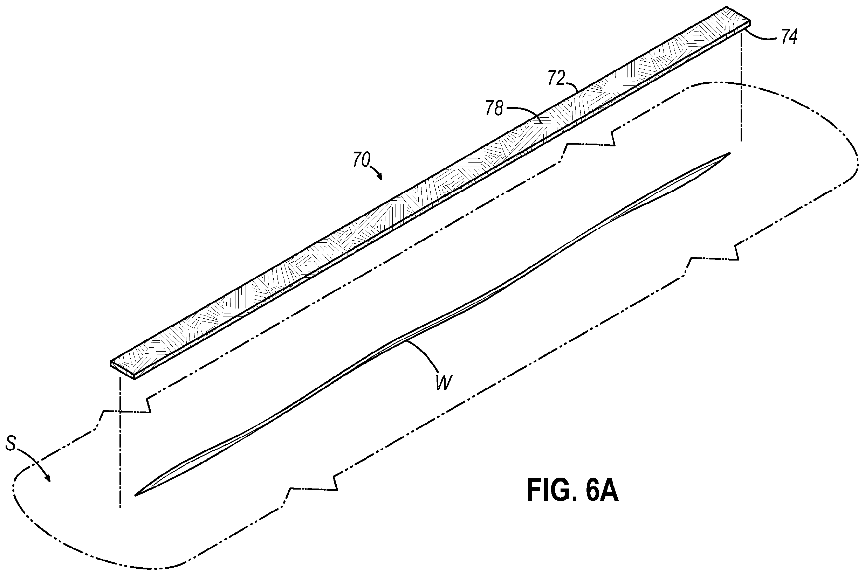

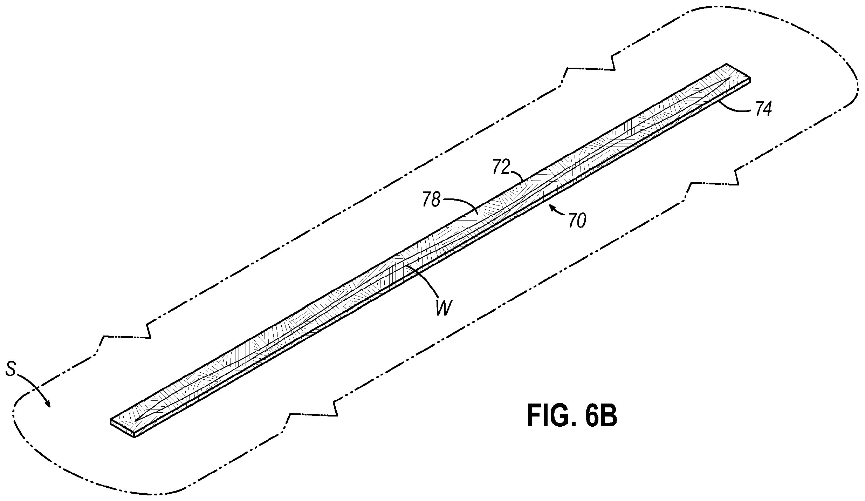

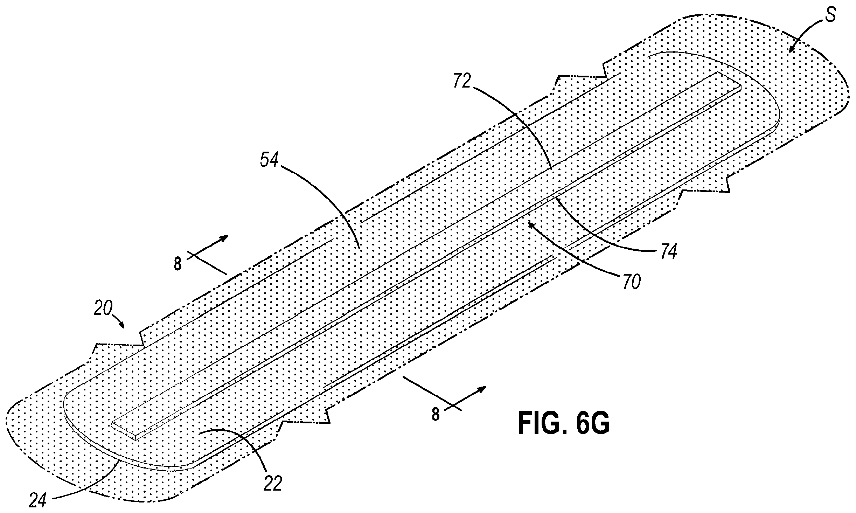

FIGS. 6A-6G show an illustrative use of wound closure system (100) having localized active strip (70). First, as shown in FIG. 6A, localized active strip (70) may be placed above wound (W) such that an imaginary centerline of active strip (70) is aligned longitudinally with edges of wound (W). Next, as shown in FIG. 6B, active strip (70) is then applied to patient skin (S) over wound (W) and the surgeon applied pressure to active strip (70) to force pressure sensitive adhesive (76) to adhere to the skin (S). In some instances, pressure sensitive adhesive (76) adheres active strip (70) to skin on one lateral edge of wound (W), and not the opposing lateral edge of wound (W).

In some instances, pressure sensitive adhesive (76) adherers active strip (70) to skin (S) on both lateral edges of wound (W). In the current example strip (70) is shown placed directly on top of wound (W). In some instances, strip (70) is located adjacent to wound (W), but entirely on one lateral side of wound (W) such that strip (70) is not directly on top of wound (W) but sufficiently close to wound (W) such that API (78) timely migrate and/or diffuse onto/into wound (W). In some instances, strip (70) does not extend the entire length of wound (W). Therefore, it should be understood, strip (70) may be placed relative to wound (W) at any suitable location as would be apparent to one skilled in the art in view of the teachings herein.

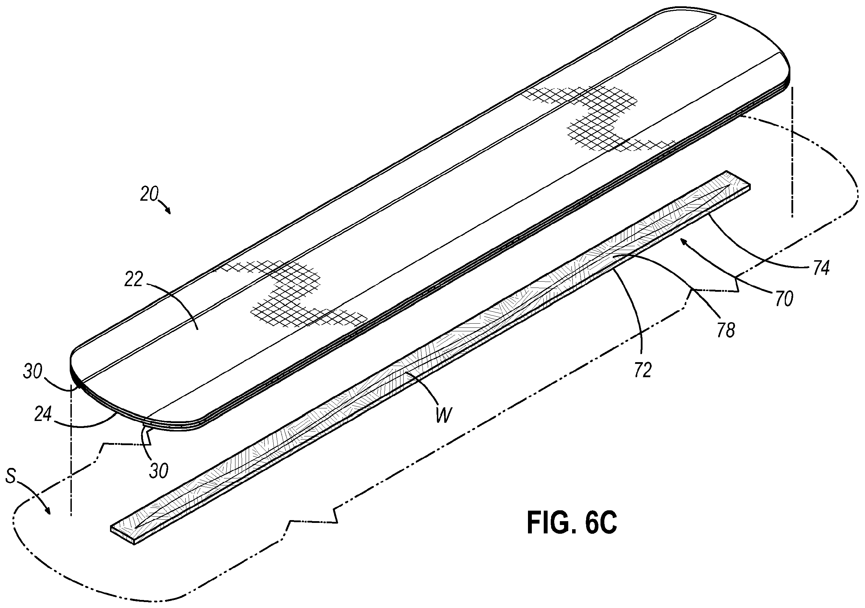

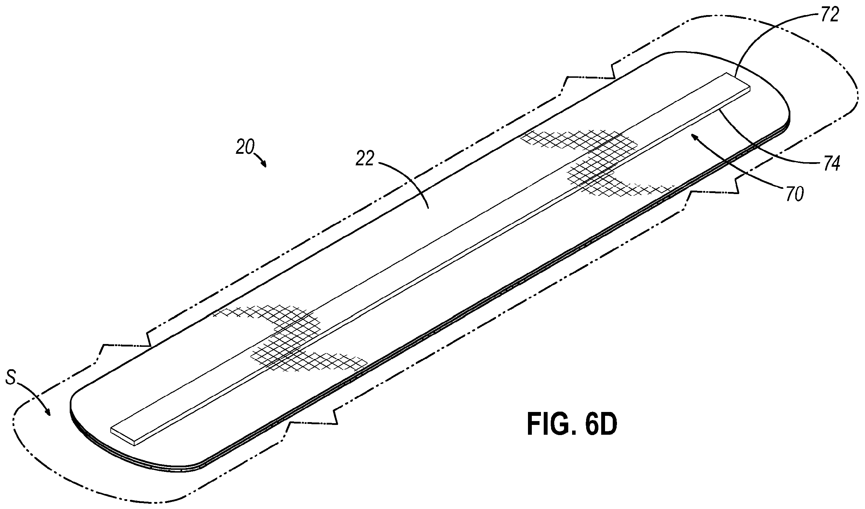

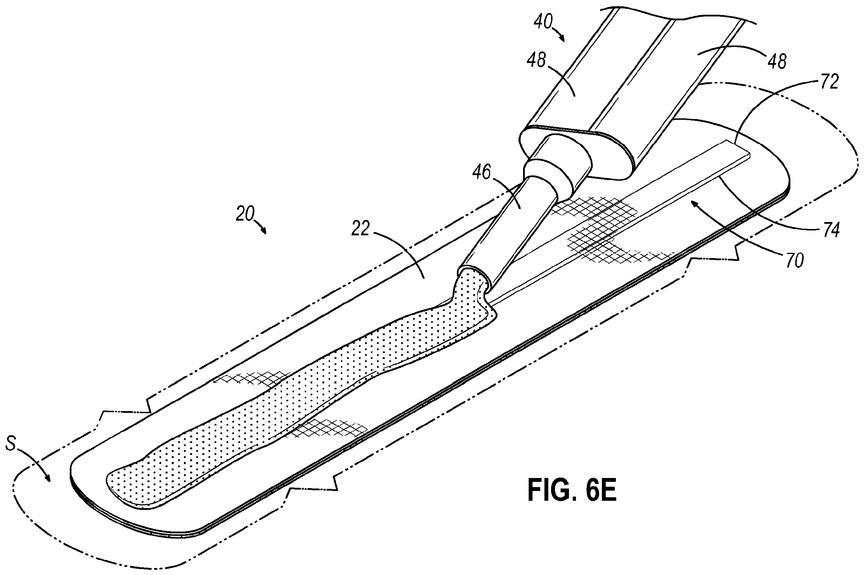

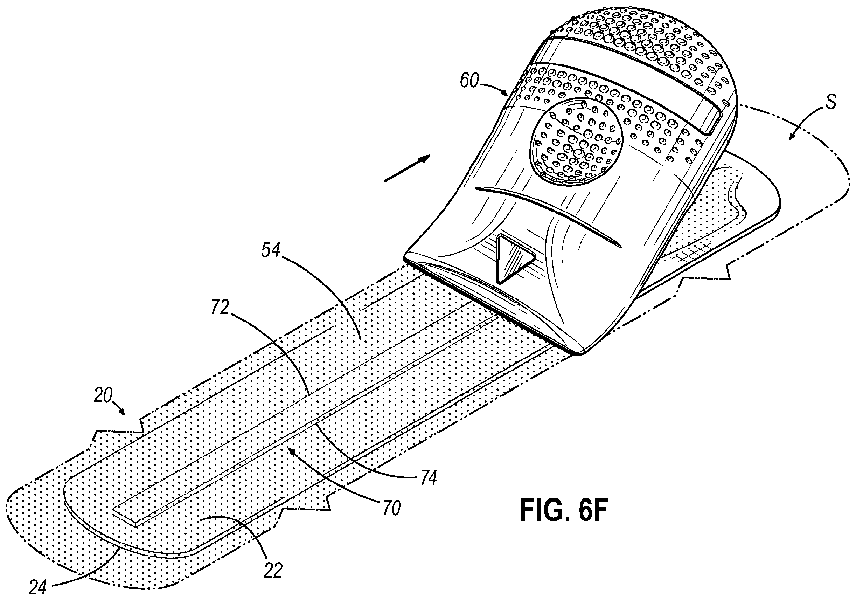

With active strip (70) suitably self-adhered to skin (S), as shown in FIG. 6C, wound closure device (20) placed above wound (W) and is aligned longitudinally with the edges of wound (W). As shown in FIG. 6D, wound closure device (20) is then applied to the patient skin (S) over both active strip (70) and wound (W); the surgeon then applies pressure to the central portion of mesh (22) to force pressure sensitive adhesive (24) to adhere to the skin (S), thus fixing the edges of wound (W) relative to one another. Wound closure device (20) may be applied in substantially similar manner described above, except now patch (20) covers both active strip (70) and wound (W). Next, as shown in FIG. 6E, adhesive applicator (40) is then used to apply a pattern of topical skin adhesive (54) to the upper side of mesh (22) of the applied wound closure device (20). As shown in FIGS. 6F-6G, and in substantially similar manner to that shown in FIGS. 3E-3F, adhesive spreader (60) is then used to spread the applied topical skin adhesive (54) uniformly over wound closure device (20) to force the topical skin adhesive (54) through the layers of mesh (22) and pressure sensitive adhesive (24) and directly against wound (W) and the surrounding skin (S). As mentioned above, elongated body (72) may at least, in some degree, inhibit topical skin adhesive (54) from interposing itself between underside (74) of strip (70) and adjacent portions of skin (S) and wound (W). In some instances, topical skin adhesive (54) may reach underside (74) of strip (70) such that about 5% to about 50% of the area of underside (74) is exposed to topical skin adhesive (54). Therefore, in some instances 95% to about 50% of the underside (74) of strip (70) is exposed to directly adjacent portions of wound (W) and skin (S).

After a suitable amount of time, such as 1-3 minutes, topical skin adhesive (54) cures, thereby forming a composite microbial barrier over wound (W), as shown in FIG. 8. With a composite microbial barrier over wound (W) formed, localized active strip (70) is directly adjacent to wound (W) and contained within the composite microbial barrier. Therefore, API (78) associated with active strip (70) may migrate and/or diffuse away from active strip (70) onto/into wound (W), thereby providing desired therapeutic effects. It should be understood, that since width (73) of strip (70) is narrower compared to patch (20) and/or cured adhesive (54), lateral edges of strip (70) are located closer to wound (W) compared to lateral edges of patch and/or cured adhesive (54). Therefore, API (78) associated with lateral ends of strip (70) may diffuse toward wound (W) within a suitable time frame to achieve the desired therapeutic effect as wound (W) heals.

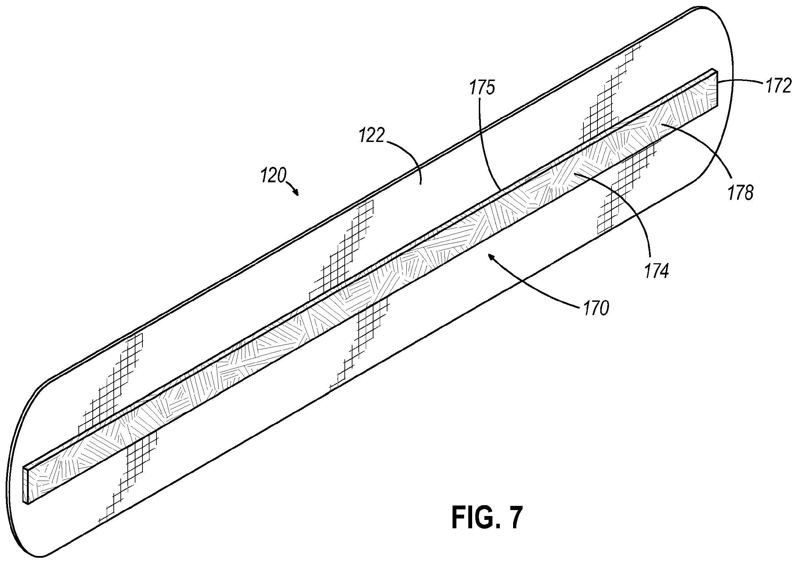

Wound closure system (100) described above includes localized active strip (70) and wound closure device (20) as separate components that were attached to skin (S) of patient sequentially. However, in some instances, localized active strip (70) and wound closure device (20) may be attached to skin (S) of patient simultaneously. FIG. 7 shows an illustrative wound closure device (120) that may be readily incorporated into wound closure system (10, 100) described above, in replacement of wound closure device (20) or in replacement of a combination of wound closure device (20) and localized active strip (70).

Wound closure device (120) includes a textile mesh (122) and a localized active strip (170); which are substantially similar to textile mesh (22) and localized active strip (70) described above, with differences elaborated below. While not shown, textile mesh (122) may include pressure sensitive adhesive located on its underside in substantially similar manner to textile mesh (22) and pressure sensitive adhesive (24) described above.

Localized active strip (170) includes an elongated body (172) with API (178) incorporated therein such that elongated body (172) and API (178) may be substantially similar to elongated body (72) and API (78) described above, with differences elaborated herein. Elongated body (172) includes an upper side (175) that is self-adhered onto the underside of textile mesh (122); while an underside (174) of elongated body (172) is lustily similar to underside (74) of elongated body (72) described above, with differences herein. Localized active strip (170) extends along an imaginary longitudinal centerline of mesh (122).

Upper side (175) of elongated body (172) may be configured to selectively attach to the underside of mesh (122) prior the illustrative use. For example, upper side (175) may include its own pressure sensitive adhesive allowing a surgeon to fix elongated body (172) to mesh (122) in preparation for illustrative use in accordance with the description herein. Alternatively, localized active strip (170) may be affixed to mesh (122) during the manufacturing process such that wound strip (170) and mesh (122) are originally presented to the surgeon as shown in FIG. 7. Regardless, with localized active strip (170) attached to the underside of mesh (122), a surgeon may align and attach mesh and activate strip (170) onto skin (S) and wound (W) in accordance with the description herein simultaneously, rather than sequentially as shown in FIGS. 6A-66D.

As mentioned above, and as shown in FIG. 6F, adhesive spreader (60) is used to spread the applied topical skin adhesive (54) uniformly over wound closure device (20) to force the topical skin adhesive (54) through the layers of mesh (22) and pressure sensitive adhesive (24) and directly against wound (W) and the surrounding skin (S). As also mentioned above, in some instances, while topical skin adhesive (54) is being force through layer of mesh (22) and/or pressure sensitive adhesive (24), adhesive (54) may cure between underside (74) of localized active strip (70) and wound (W). If adhesive (54) cures between strip (70) and wound (W), API (78) may be undesirably inhibited from diffusing/migrating into wound (W). Therefore, it may be desirable to further inhibit topical skin adhesive (54) from curing between underside (74) of localized active strip (70) and wound (W) in order to promote suitable diffusion/migration of API (78) into wound (W).

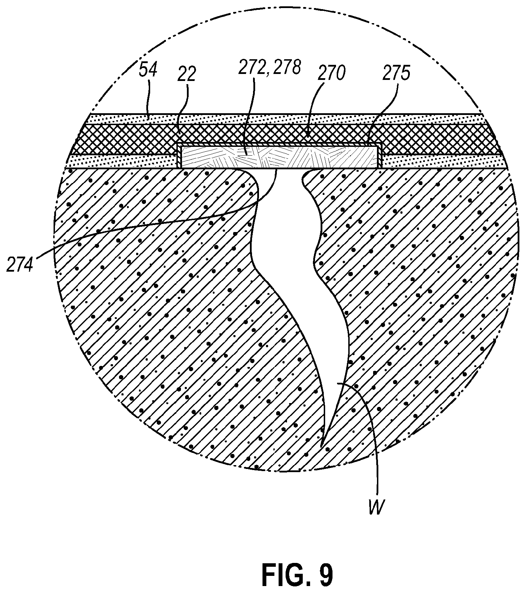

FIG. 9 shows an alternative localized active strip (270) configured to inhibit topical skin adhesive (54) from curing between the underside (274) of strip (270) and wound (W) in order to promote suitable diffusion/migration of API (278) into wound (W). Localized active strip (270) may be readily incorporated into wound closure system (100) and/or wound closure device (120) described above in replacement of localized active strip (70, 170) described above respectively. Localized active strip (270) is substantially similar to localized active strip (70, 170) described above, with differences elaborated herein. Therefore, localized active strip (270) includes an elongated body (272) having API (278) incorporated therein, and an underside (274) configured to be directly adjacent to wound (W) when suitably deployed in accordance with the description herein; which are substantially similar to elongated body (72, 172), API (78, 178), and underside (74, 174) described above, respectively, with differences elaborated herein.

Additionally, localized active strip (270) also includes a protective element in the form of a mask (275). Mask (275) is located adjacent to an upper surface of elongated body (272). Mask (275) is formed of a suitable material configured to inhibit uncured topical skin adhesive (54) from penetrating through mask (275) while adhesive (54) is applied in accordance with the description herein. In some instances, mask (275) may be impervious to topical skin adhesive (54); while in other instances, mask (275) may be configured to suitably inhibit topical skin adhesive (54) from reaching underside (274) prior to curing. Therefore, while adhesive spreader (60) pushes topical skin adhesive (54) through portions of mesh (22), mask (275) may act as a sheath surrounding the top surface of strip (270), thereby inhibiting topical skin adhesive (54) from reaching underside (274) of strip (270). With mask (275) being located above the top surface of elongated body (272), mask (275) is configured to inhibit adhesive (54) from reaching and curing at a location interposed between wound (W) and underside (274) of elongated body (272). As such, mask (275) may help ensure API (278) suitably migrates/diffuses from elongated body (272) into wound (W) in accordance with the description herein.

While the current embodiment shows mask (275) being directly on top of the upper surface of elongated body (272), this is merely optional. In some examples, mask (275) may vertically offset from the top surface of elongated body (272). While mask (275) is shown as a component of localized active strip (270), it should be understood mask (275) may be its own component, associated with mesh (22), or associated with any other suitable component as would be apparent to one skilled in the art in view of the teachings herein.

Mask (275) may extend longitudinally along the entire length of elongated body (272). Alternatively, mask (275) may extend along a substantial length of elongated body (272). As another illustrative alternative, mask (275) may include a longitudinal array of segments extending along the entire or partial length of elongated body (272).

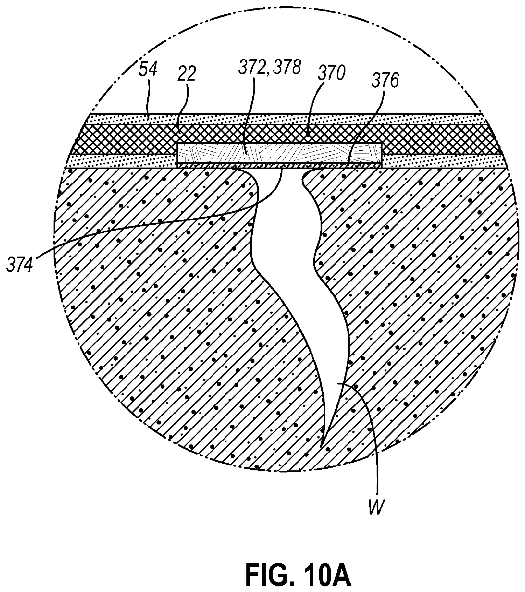

FIGS. 10A-10B show an alternative localized active strip (370) configured to inhibit topical skin adhesive (54) from curing between the underside (374) of strip (370) and wound (W) in order to promote suitable diffusion/migration of API (378) into wound (W). Localized active strip (370) may be readily incorporated into wound closure system (100) and/or wound closure device (120) described above in replacement of localized active strip (70, 170, 270) described above respectively. Localized active strip (370) is substantially similar to localized active strip (70, 170, 270) described above, with differences elaborated herein. Therefore, localized active strip (370) includes an elongated body (372) having API (378) incorporated therein, and an underside (374) configured to be directly adjacent to wound (W) when suitably deployed in accordance with the description herein; which are substantially similar to elongated body (72, 172, 272), API (78, 178, 278), and underside (74, 174, 274) described above, respectively, with differences elaborated herein.

Rather than having a mask (275) to inhibit curing of adhesive (54) between underside (374) of strip (370) and wound (W), localized active strip (370) includes a soluble pressure sensitive adhesive (376) spanning longitudinally along a substantial portion of underside (374). Pressure sensitive adhesive (376) allows a surgeon to self-adhere strip (370) onto skin (S) of wound (W) in substantially similar fashion to pressure sensitive adhesive (24, 76) described above.

Additionally, as shown in FIG. 10A, pressure sensitive adhesive (376) functions as a sheath while topical skin adhesive (54) is spread with adhesive spreader (60) in accordance with the description herein. Therefore, while adhesive spreader (60) pushes adhesive (54) through portions of mesh (22) and/or elongated body (372), the layer of pressure sensitive adhesive (376) may act as a sheath interposed between underside (374) of strip (370) and skin (S), thereby inhibiting topical skin adhesive (54) from reaching the portion of skin directly adjacent to underside (374) of strip (370). With pressure sensitive adhesive (376) being located between skin (S) and underside (374) of strip (370), pressure sensitive adhesive (376) is configured to inhibit topical skin adhesive (54) from reaching and curing at a location interposed between wound (W) and underside (374) of elongated body (372).

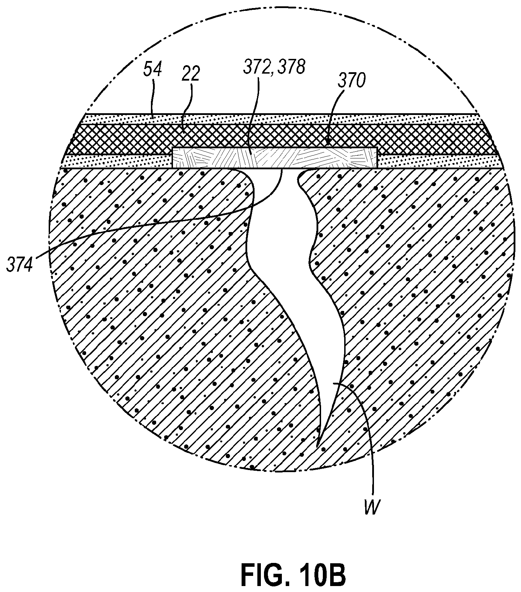

However as mentioned above, pressure sensitive adhesive (376) is also soluble. Therefore, as shown in FIG. 10B, a suitable amount of time after topical skin adhesive (54) cures, pressure sensitive adhesive (376) is configured to dissolve in response to exposure to fluids. With pressure sensitive adhesive (376) dissolved, API (378) is free to migrate/diffuse from elongated body (372) into wound (W). As such, pressure sensitive adhesive (376) may help ensure API (378) suitably migrates/diffuses from elongated body (272) into wound (W) in accordance with the description herein.

### V. Miscellaneous

It is understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those of ordinary skill in the art in view of the teachings herein.

Versions of the devices described above may have application in conventional medical treatments and procedures conducted by a medical professional, as well as application in robotic-assisted medical treatments and procedures. By way of example only, various teachings herein may be readily incorporated into a robotic surgical system such as the DA VINCI^{®} system by Intuitive Surgical, Inc., of Sunnyvale, California.

Versions of the devices described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by a user immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK^{®} bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

Having shown and described various embodiments of the present invention, further adaptations of the methods and systems described herein may be accomplished by appropriate modifications by one of ordinary skill in the art without departing from the scope of the present invention. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A skin closure system, comprising:
(a) a surgical mesh including a first upper side and a first lower side, wherein the first lower side is configured to adhere to a section of skin of a patient that surrounds a wound such that the surgical mesh surrounds the wound, wherein the first lower side of the surgical mesh comprises a first area;
(b) a topical skin adhesive configured to be applied to the first upper side of the surgical mesh, wherein the topical skin adhesive is configured to cure within the surgical mesh to thereby form a protective layer over the wound; and
(c) a strip configured to be secured relative to the surgical mesh, the strip comprising:
(i) an elongated body comprising a second upper side and a second lower side, wherein the second lower side comprises a second area, wherein the second area of the second lower side of the elongated body is smaller than the first area of the first lower side of the surgical mesh, wherein the elongated body is configured to extend longitudinally along a central portion of the lower side of the elongated mesh while the surgical mesh surrounds the wound such that the elongated body is adjacent to the wound, and
(ii) an active pharmaceutical ingredient integrated into the elongated body, wherein the active pharmaceutical ingredient is configured to diffuse from the elongated body toward the wound after the protective layer over the wound is formed.

2. The skin closure system of claim 1, wherein the second area of the second lower side of the elongated body is between 5 percent and 30 percent of the first area of the first lower side of the surgical mesh.

3. The skin closure system of any one of claims 1-2, wherein the elongated body is configured to inhibit penetration of the topical skin adhesive through the elongated body to the second lower side of the elongated body.

4. The skin closure system of any one of claims 1-3, wherein the surgical mesh comprises a first porosity, wherein the elongated body comprises a second porosity, wherein the second porosity is lower than the first porosity.

5. The skin closure system of any one of claims 1-3, wherein the elongated body comprises a material that is impervious to penetration of the topical skin adhesive.

6. The skin closure system of any one of claims 1-5, wherein the surgical mesh comprises a first width, wherein the elongated body comprises a second width, wherein the second width is smaller than the first width.

7. The skin closure system of any one of claims 1-6, further comprising a mask interposed between the second upper surface of the elongated body and the first upper surface of the surgical mesh.

8. The skin closure system of claim 7, wherein the mask is directly on top of the second upper surface of the elongated body, wherein the mask extends longitudinally along the entire length of the elongated body.

9. The skin closure system of claim 7, wherein the mask comprises a material that is impervious to penetration of the topical adhesive.

10. The skin closure system of any one of claims 1-9, wherein the strip further comprises a pressure sensitive adhesive associated with the second lower side of the elongated body.

11. The skin closure system of claim 10, wherein the pressure sensitive adhesive is disposed on the second lower side of the elongated body in a stripe pattern.

12. The skin closure system of any one of claims 1-11, wherein the surgical mesh comprises polyethylene.

13. The skin closure system of any one of claims 1-12, wherein the active pharmaceutical ingredient comprises triclosan.

14. The skin closure system of any one of claims 1-13, wherein the elongated body comprises polyethylene terephthalate.

15. A method of making the skin closure system of claim 1, wherein the active pharmaceutical ingredient comprises triclosan, the method comprising:
(a) positioning the elongated body and a source reservoir within a sealed pouch, wherein the source reservoir comprises triclosan;
(b) positioning the sealed pouch within a chamber; and
(c) subjecting the chamber to an ethylene oxide sterilization process and thereby urging the triclosan to migrate from the source reservoir to the elongated body.

## Patentansprüche

1. Hautverschlusssystem, umfassend:
(a) ein chirurgisches Netz, das eine erste Oberseite und eine erste Unterseite einschließt, wobei die erste Unterseite konfiguriert ist, um an einem Abschnitt der Haut eines Patienten zu haften, der eine Wunde umgibt, sodass das chirurgische Netz die Wunde umgibt, wobei die erste Unterseite des chirurgischen Netzes einen ersten Bereich umfasst;
(b) einen topischen Hautklebstoff, der konfiguriert ist, um auf die erste Oberseite des chirurgischen Netzes aufgebracht zu werden, wobei der topische Hautklebstoff konfiguriert ist, um innerhalb des chirurgischen Netzes auszuhärten, um dadurch eine Schutzschicht über der Wunde zu bilden; und
(c) einen Streifen, der konfiguriert ist, um relativ zu dem chirurgischen Netz befestigt zu werden, der Streifen umfassend:
(i) einen länglichen Körper, umfassend eine zweite Oberseite und eine zweite Unterseite, wobei die zweite Unterseite einen zweiten Bereich umfasst, wobei der zweite Bereich der zweiten Unterseite des länglichen Körpers kleiner ist als der erste Bereich der ersten Unterseite des chirurgischen Netzes, wobei der längliche Körper konfiguriert ist, um sich in Längsrichtung entlang eines mittleren Abschnitts der Unterseite des länglichen Netzes zu erstrecken, während das chirurgische Netz die Wunde umgibt, sodass der längliche Körper der Wunde benachbart ist, und
(ii) einen pharmazeutischen Wirkstoff, der in den länglichen Körper integriert ist, wobei der pharmazeutische Wirkstoff konfiguriert ist, um von dem länglichen Körper in Richtung der Wunde zu diffundieren, nachdem die Schutzschicht über der Wunde gebildet worden ist.

2. Hautverschlusssystem nach Anspruch 1, wobei der zweite Bereich der zweiten unteren Seite des länglichen Körpers zwischen 5 Prozent und 30 Prozent des ersten Bereichs der ersten unteren Seite des chirurgischen Netzes beträgt.

3. Hautverschlusssystem nach einem der Ansprüche 1 bis 2, wobei der längliche Körper konfiguriert ist, um das Eindringen des topischen Hautklebstoffs durch den länglichen Körper zur zweiten Unterseite des länglichen Körpers zu verhindern.

4. Hautverschlusssystem nach einem der Ansprüche 1 bis 3, wobei das chirurgische Netz eine erste Porosität umfasst, wobei der längliche Körper eine zweite Porosität umfasst, wobei die zweite Porosität geringer ist als die erste Porosität.

5. Hautverschlusssystem nach einem der Ansprüche 1 bis 3, wobei der längliche Körper ein Material umfasst, das für das Eindringen des topischen Hautklebstoffs undurchlässig ist.

6. Hautverschlusssystem nach einem der Ansprüche 1 bis 5, wobei das chirurgische Netz eine erste Breite umfasst, wobei der längliche Körper eine zweite Breite umfasst, wobei die zweite Breite kleiner ist als die erste Breite.

7. Hautverschlusssystem nach einem der Ansprüche 1 bis 6, ferner umfassend eine Maske, die zwischen der zweiten oberen Oberfläche des länglichen Körpers und der ersten oberen Oberfläche des chirurgischen Netzes angeordnet ist.

8. Hautverschlusssystem nach Anspruch 7, wobei die Maske direkt auf der zweiten oberen Oberfläche des länglichen Körpers liegt, wobei sich die Maske in Längsrichtung über die gesamte Länge des länglichen Körpers erstreckt.

9. Hautverschlusssystem nach Anspruch 7, wobei die Maske ein Material umfasst, das für das Eindringen des topischen Klebstoffs undurchlässig ist.

10. Hautverschlusssystem nach einem der Ansprüche 1 bis 9, wobei der Streifen ferner einen druckempfindlichen Klebstoff umfasst, der mit der zweiten Unterseite des länglichen Körpers verbunden ist.

11. Hautverschlusssystem nach Anspruch 10, wobei der druckempfindliche Klebstoff auf der zweiten Unterseite des länglichen Körpers in einem Streifenmuster angeordnet ist.

12. Hautverschlusssystem nach einem der Ansprüche 1 bis 11, wobei das chirurgische Netz Polyethylen umfasst.

13. Hautverschlusssystem nach einem der Ansprüche 1 bis 12, wobei der aktive pharmazeutische Wirkstoff Triclosan umfasst.

14. Hautverschlusssystem nach einem der Ansprüche 1 bis 13, wobei der längliche Körper Polyethylenterephthalat umfasst.

15. Verfahren zur Herstellung des Hautverschlusssystems nach Anspruch 1, wobei der pharmazeutische Wirkstoff Triclosan umfasst, das Verfahren umfassend:
(a) Positionieren des länglichen Körpers und eines Quellenreservoirs innerhalb eines versiegelten Beutels, wobei das Quellenreservoir Triclosan umfasst;
(b) Positionieren des versiegelten Beutels in einer Kammer; und
(c) Unterziehen der Kammer einem Ethylenoxidsterilisationsprozess, wodurch das Triclosan aus dem Quellenreservoir in den länglichen Körper wandert.

## Revendications

1. Système de fermeture cutanée comprenant :
a) un treillis chirurgical comportant un premier côté supérieur et un premier côté inférieur, dans lequel le premier côté inférieur est conçu pour adhérer à une section de peau d'un patient qui entoure une plaie de telle sorte que le treillis chirurgical entoure la plaie, dans lequel le premier côté inférieur du treillis chirurgical comprend une première zone ;
(b) un adhésif cutané topique conçu pour être appliqué sur le premier côté supérieur du treillis chirurgical, dans lequel l'adhésif cutané topique est conçu pour durcir à l'intérieur du treillis chirurgical pour former ainsi une couche protectrice sur la plaie ; et
c) une bande conçue pour être fixée par rapport au treillis chirurgical, la bande comprenant :
i) un corps allongé comprenant un second côté supérieur et un second côté inférieur, dans lequel le second côté inférieur comprend une seconde zone, dans lequel la seconde zone du second côté inférieur du corps allongé est plus petite que la première zone du premier côté inférieur du treillis chirurgical, dans lequel le corps allongé est conçu pour s'étendre longitudinalement le long d'une partie centrale du côté inférieur de la maille allongée lorsque le treillis chirurgical entoure la plaie de telle sorte que le corps allongé est adjacent à la plaie, et
(ii) un ingrédient pharmaceutique actif intégré dans le corps allongé, dans lequel l'ingrédient pharmaceutique actif est conçu pour diffuser du corps allongé vers la plaie après la formation de la couche protectrice sur la plaie.

2. Système de fermeture cutanée selon la revendication 1, dans lequel la seconde zone du second côté inférieur du corps allongé est comprise entre 5 % et 30 % de la première zone du premier côté inférieur du treillis chirurgical.

3. Système de fermeture cutanée selon l'une quelconque des revendications 1 à 2, dans lequel le corps allongé est conçu pour inhiber la pénétration de l'adhésif cutané topique à travers le corps allongé jusqu'au second côté inférieur du corps allongé.

4. Système de fermeture cutanée selon l'une quelconque des revendications 1 à 3, dans lequel le treillis chirurgical comprend une première porosité, dans lequel le corps allongé comprend une seconde porosité, dans lequel la seconde porosité est inférieure à la première porosité.

5. Système de fermeture cutanée selon l'une quelconque des revendications 1 à 3, dans lequel le corps allongé comprend un matériau qui est imperméable à la pénétration de l'adhésif cutané topique.

6. Système de fermeture cutanée selon l'une quelconque des revendications 1 à 5, dans lequel le treillis chirurgical comprend une première largeur, dans lequel le corps allongé comprend une seconde largeur, dans lequel la seconde largeur est plus petite que la première largeur.

7. Système de fermeture cutanée selon l'une quelconque des revendications 1 à 6, comprenant en outre un masque interposé entre la seconde surface supérieure du corps allongé et la première surface supérieure du treillis chirurgical.

8. Système de fermeture cutanée selon la revendication 7, dans lequel le masque est directement au-dessus de la seconde surface supérieure du corps allongé, dans lequel le masque s'étend longitudinalement sur toute la longueur du corps allongé.

9. Système de fermeture cutanée selon la revendication 7, dans lequel le masque comprend un matériau qui est imperméable à la pénétration de l'adhésif topique.

10. Système de fermeture cutanée selon l'une quelconque des revendications 1 à 9, dans lequel la bande comprend en outre un adhésif sensible à la pression associé au second côté inférieur du corps allongé.

11. Système de fermeture cutanée selon la revendication 10, dans lequel l'adhésif sensible à la pression est disposé sur le second côté inférieur du corps allongé dans un motif de rayures.

12. Système de fermeture cutanée selon l'une quelconque des revendications 1 à 11, dans lequel le treillis chirurgical comprend du polyéthylène.

13. Système de fermeture cutanée selon l'une quelconque des revendications 1 à 12, dans lequel l'ingrédient pharmaceutique actif comprend du triclosan.

14. Système de fermeture cutanée selon l'une quelconque des revendications 1 à 13, dans lequel le corps allongé comprend du polyéthylène téréphtalate.

15. Procédé de fabrication du système de fermeture cutanée selon la revendication 1, dans lequel l'ingrédient pharmaceutique actif comprend du triclosan, le procédé comprenant :
a) le positionnement du corps allongé et d'un réservoir source à l'intérieur d'une poche scellée, dans lequel le réservoir source comprend du triclosan ;
b) le positionnement de la pochette scellée à l'intérieur d'une chambre ; et
c) la soumission de la chambre à un procédé de stérilisation à l'oxyde d'éthylène et poussant ainsi le triclosan à migrer du réservoir source vers le corps allongé.
